# EUROPEAN PATENT APPLICATION

(11) **EP 1 829 488 A1**
(43) Date of publication of application: **05.09.2007**
(21) Application number: 06380041.1
(22) Date of filing: 01.03.2006
(51) Int. Cl.: A61B 17/02

(54) **Improved retractor for surgical use**

(71) Applicant: ANSABERE SURGICAL S.L., 31110 Noain (Navarra) (ES)
(72) Inventor: Insausti Isturiz, Jesus Maria, 31110 Noain Navarra (ES)
(74) Representative: Isern-Jara, Nuria

(57) **Abstract**

An improved retractor for surgical use, particularly for fitting into an adaptable surgical support, has a plurality of elongated clamshell like elements that move in relation to each other, which includes two elongated adjacent arms (1 and 2) that are connected to each other by means of an interconnecting segment, each one of the elongated arms (1 and 2) being made up by a first section (6) in the form of a handle and a second elongated section (7) at the end of which a perpendicular extension is fitted in the form of a elongated clamshell like element (9), there also being a bridging element (10) located between said arms and fitted to same, a third perpendicular extension protruding from said bridge (10) in the shape of a third elongated clamshell like element (11) with adjustable longitudinal movement with a toothed wheel (13), so that in the working position of the retractor, the patient's wall onto which said retractor is acting has three points of contact coinciding with the three elongated clamshell like elements (9 and 11).

## Description

### OBJECT OF THE INVENTION

The object of the present Invention is an improved retractor for surgical use that includes significant innovations and advantages compared to other retractors or pieces of equipment currently in use for the same purpose.

More specifically it refers to an improved retractor for surgical use, and particularly to be fitted into a surgical support, which has a plurality of easy use articulated elongated clamshell like elements which are moveable in relation to each other.

### BACKGROUND TO THE INVENTION

At the present time the method or system used to carry out the separation and stabilisation in the surgical area of the cavity or incision made into the body of a patient during a surgical operation is the use of manual fixing elongated clamshell like elements, which have to be held by the surgeons themselves with the disadvantage that this means to proceed simultaneously with the surgical operation.

Other pieces of equipment are also known, such as anoscopes, used for anal opening and which are made up of two interconnected elongated clamshell like elements, nevertheless, they are of little practical use in surgery as said elements are usually very wide and occupy a great deal of the wall preventing access to same.

In the same way, other pieces of retractor equipment are also known with greater complexity which attempt to solve the above-mentioned disadvantages; however, they are very expensive and require the use of specific additional training for the user, in this case, the surgeon.

These types of small incisions require the surgeon to be placed very close to the incision, thus making the conventional systems of lighting very uncomfortable and insufficient.

### DESCRIPTION OF THE INVENTION

The present invention has been developed for the purpose of providing a surgical retractor that solves the previously stated disadvantages, moreover, providing other additional advantages that will be clear from the description which is attached below.

The improved retractor for surgical use of this present invention, particularly for fitting into an adaptable surgical support, has a plurality of elongated clamshell like elements that move in relation to each other, and is characterised because of the fact that it includes two elongated adjacent arms that are connected to each other by means of an interconnecting segment, each one of the elongated arms being made up by a first section in the form of a handle and a second elongated section at the end of which a perpendicular extension is fitted in the form of a elongated clamshell like element, there also being a bridging element located between said arms and fitted to same, a third perpendicular extension protruding from said bridge in the shape of a third elongated clamshell like element with adjustable movement with a means of regulation, so that in the working position of the retractor, the patient's wall onto which said retractor is acting has three points of contact coinciding with the three elongated clamshell like elements.

Thanks to these characteristics, a retractor is obtained with a simplified mechanism that allows total visualisation of the surgical field and easy handling and less cost in comparison with the retractors of earlier techniques, allowing the surgeon to carry out the operation without the need to support the said retractor by hand.

Said retractor is very suitable for trans-anal operations (recto-anal tumours and fistula surgery), for vertebral surgery and gynaecological surgery via the vagina.

In accordance with another aspect of the invention, the means of regulation are made up by a toothed wheel housed in the bridge element that is fitted into a toothed rack that can be moved axially in both directions, said rack being fitted at one end of the third perpendicular extension. In addition, said toothed wheel has a locking mechanism to prevent the voluntary turning movement of same.

By preference, the elongated clamshell shaped extension is made up of a body that has an upper portion that is adaptable to the extended section of the arms and to the central support element and a lower extended portion with an arched shape and with variable length.

The stated upper portions of the extensions of the clamshell like elements have a through orifice that ends at the interior side wall of the lower portion. In this way, lighting can be fitted to the retractor that is very suitable so that the surgeon can adequately see the working area.

Advantageously, the surgical retractor has fixing devices, both on the bridging element, and equally on the extended arms, to an adaptable surgical support.

In the preferred embodiment, the bridging element is fitted to both elongated sections of each one of the arms by means of side flanges fixed by screw or similar elements.

By means of the retractor of the invention the surgical resection of tumours can be undertaken by trans-anal means that under other circumstances would have to be undertaken through the abdomen in such a way that it could involve the amputation of the patient's rectum, compelling the patient to wear a bag fitted to the orifice made in the abdomen for the evacuation of faeces for the rest of his/her life.

Other characteristics and advantages of the surgical retractor object of the present invention will be come clear from the present description of a preferred embodiment, but it is not exclusive, it illustrates by way of example, but the drawings attached are not by way of limitation, in which:

### BRIEF DESCRIPTION OF THE DRAWINGS

Figure 1 shows a perspective view of the improved retractor for surgical use in accordance with this present invention in its rest position;
Figure 2 shows a perspective view of the improved retractor represented in the previous drawing as seen from below;
Figure 3 shows a perspective view of the elongated arms of the retractor without the elongated clamshell like elements and means for the regulation of the third elongated clamshell like element;

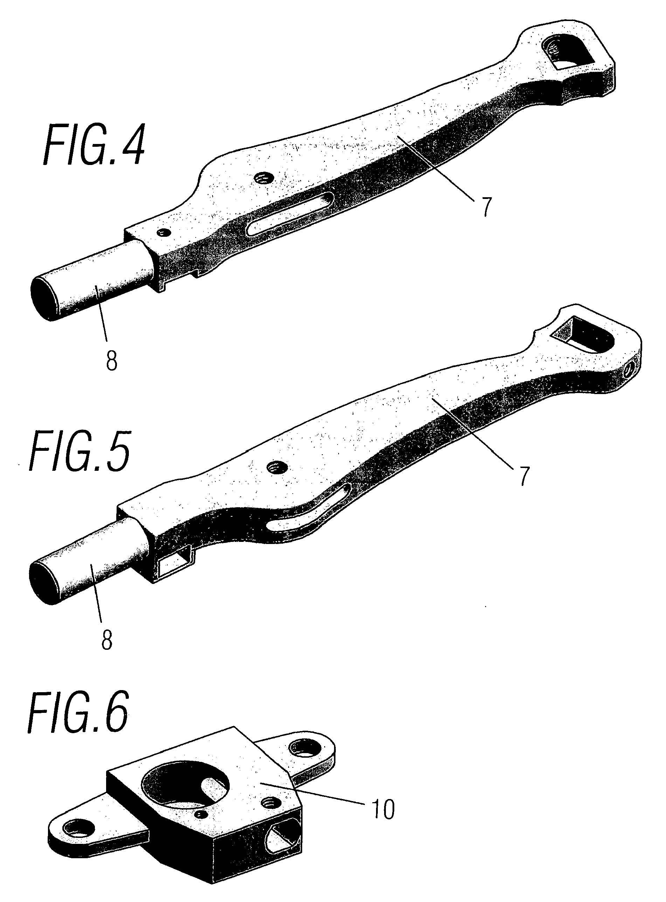

Figure 4 shows a perspective view of the left elongated arm of the retractor;
Figure 5 shows a perspective view of the right elongated arm of the retractor;
Figure 6 shows a perspective view of the bridging element;
Figure 7 shows a perspective view of the third elongated clamshell like element;
Figure 8 shows a perspective view of the elongated clamshell like element housed in the two elongated arms;
Figure 9 shows a detailed perspective view of the toothed rack for the means of regulation;
Figure 10 shows a detailed perspective view of the toothed wheel;
Figure 11 shows a detailed perspective view of the retractor according to the invention with the three elongated clamshell like elements extended.

### DESCRIPTION OF THE PREFERRED EMBODIMENT

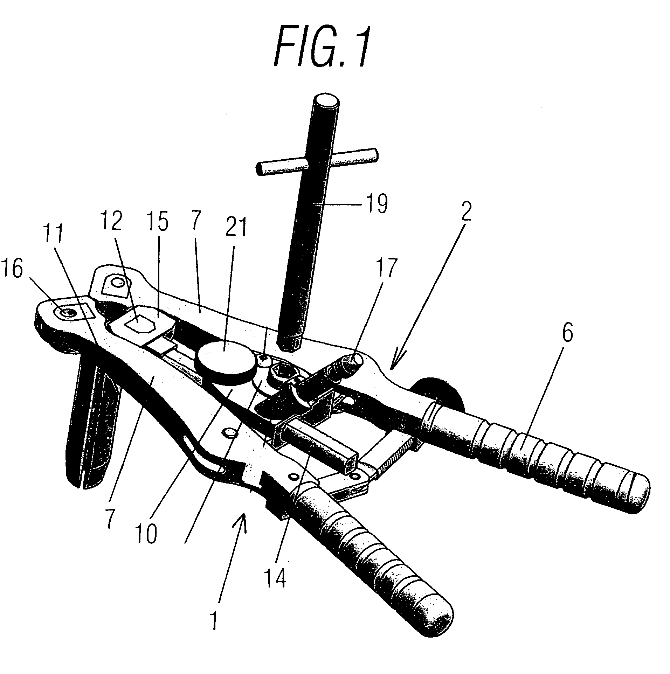

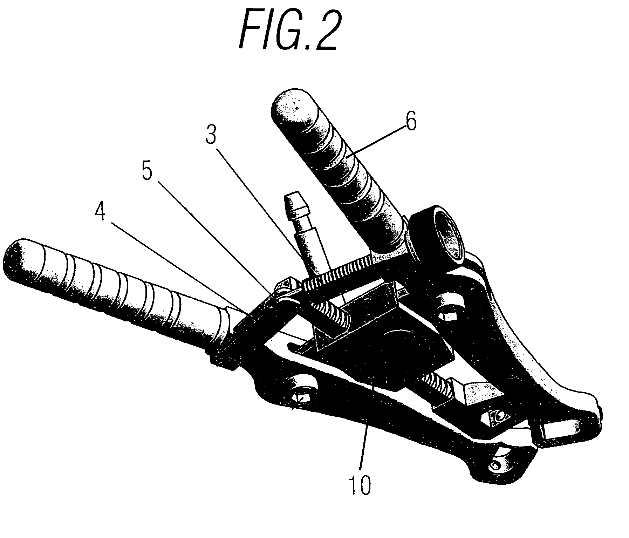

As shown in figures 1 and 2, a retractor for surgical use of the present invention, particularly to contribute to the stabilising of a specific area of the patient's body whilst carrying out trans-anal surgical operations and which can be fitted to an adaptable conventional support, it includes two adjacent elongated arms (1 and 2) connected to each other by means of an interconnecting segment made up of two bodies (3 and 4) which are articulated by means of a pin (5). One of the two bodies (3) has two opposing threaded sides, in such a way as to allow for the adjustment of its position against the other body (4) by means of using a milled knob (18).

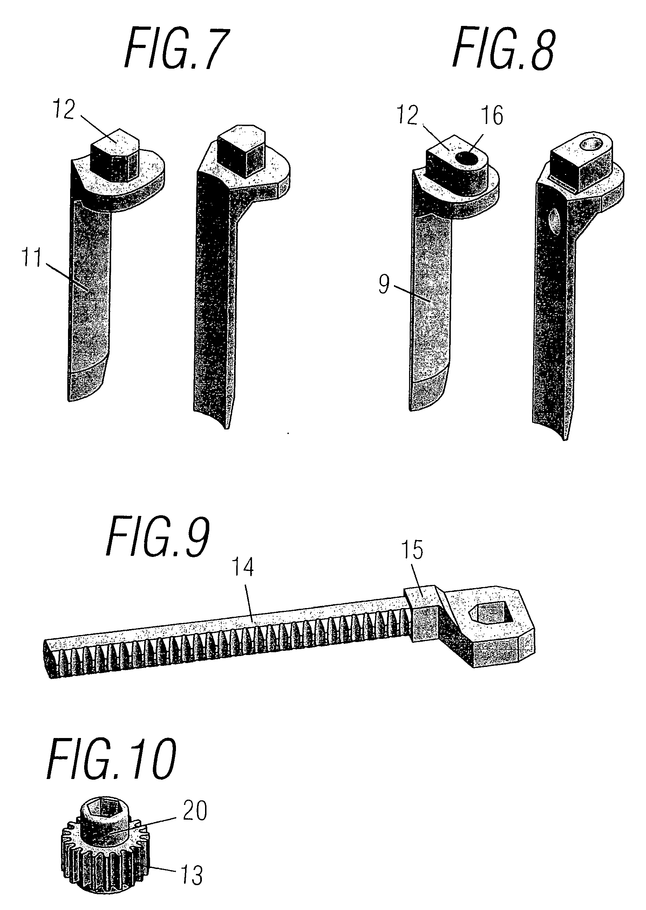

Each one of the elongated arms (1 and 2) is made up of a first section (6) in the shape of a handle for the grip and the opening of the elongated clamshell like element (9) and some second elongated sections (7) joined to said sections (6) at the end of which there is a perpendicular extension in the shape of a elongated clamshell like element (9), also having been fitted with a bridging element (10) (see a preferred non-limitative structural arrangement in Figure 6) located between said arms (1 and 2) and fitted to same, a third perpendicular elongation protruding from said bridge (10) in the shape of a third elongated clamshell like element (11) with a longitudinal movement adjustable by means of a regulator, so that in the working position of the retractor, the zone or walls of the patient on which the said retractor works has three points of contact coinciding with the three elongated clamshell shaped extension (9 and 11) which appreciably make the work of the surgeon easier.

As can be seen in figures 7 and 8, the clamshell shaped elongations (9 and 11) are basically made up of a body that has an upper section (12) with an adjustable arrangement onto the elongated arms (1 and 2) and the central support element (15) respectively and an elongated lower section with an arched shape.

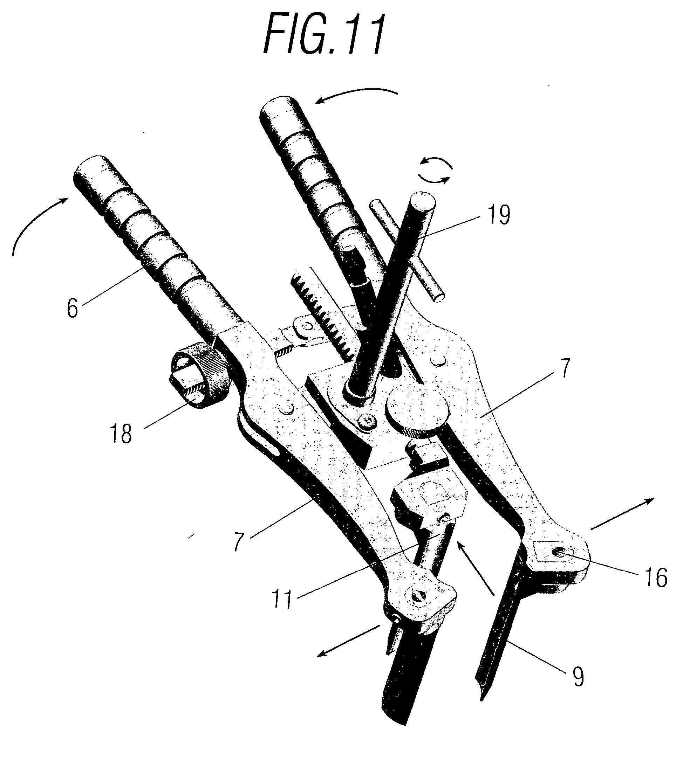

Making reference to figures 9 and 10, and especially to the previously stated means of adjustment they are made up with a toothed wheel (13) fixed in the bridging element (10) that is fitted to a toothed rack (14) axially moveable in both directions, said rack (14) being fitted at one end to the third perpendicular extension by means of a central support element (15). The toothed wheel (13) can be turned by inserting a suitable tool (19) into the slot (20) (see figure 10). On the other hand, the required positioning of the toothed rack (14) can be locked during its operation by means of a milled knob (21) equally housed in the upper part of the bridging element (10).

The fact must be highlighted that the retractor also has an advantageous means of lighting for the working area that is fitted by means of each one of the through openings (16) of the side elongated clamshell like elements (9) in such a way that the field of vision for the surgeon is excellent as there can be two points of illumination provided.

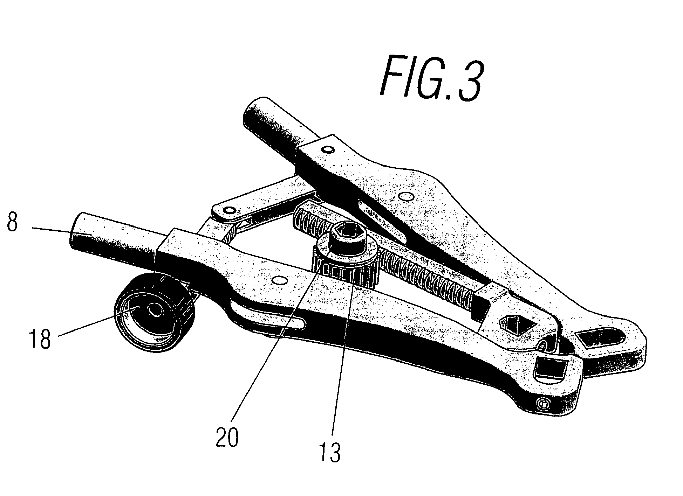

The fitting of the here described retractor into an adaptable surgical support can be carried out by means of an adaptor (17) located on the upper part of the bridging element (10) and on the protruding shafts (8) of the elongated arms (1 and 2) as can be seen in figure 3.

In figure 11 the differing movements that can be made by the different components that make up the retractor are shown by means of arrows, and in which the elongated clamshell shaped extensions (9 and 11) are arranged in an extended position, meaning their working position.

The details, shapes, sizes and other accessorial elements, likewise the materials used for the construction of the improved surgical retractor of the invention can suitably be substituted for others that are technically equivalent and do not deviate from the essentials of the invention object or the scope defined by the claims which are included below.

## Claims

1. An improved retractor for surgical use, particularly for fitting into an adaptable surgical support, has a plurality of elongated clamshell like elements that move in relation to each other, and is **characterised** because of the fact that it includes two elongated adjacent arms (1 and 2) that are connected to each other by means of an interconnecting segment, each one of the elongated arms (1 and 2) being made up by a first section (6) in the form of a handle and a second elongated section (7) at the end of which a perpendicular extension is fitted in the form of a elongated clamshell like element (9), there also being a bridging element (10) located between said arms and fitted to same, a third perpendicular extension protruding from said bridge (10) in the shape of a third elongated clamshell like element (11) with adjustable longitudinal movement with a means of regulation, so that in the working position of the retractor, the patient's wall onto which said retractor is acting has three points of contact coinciding with the three elongated clamshell like elements (9 and 11).

2. An improved retractor according to claim 1, **characterised in that** it has some means of regulation that are made up of a toothed wheel (13) housed in the bridging element (10) that is fitted with a toothed rack (14) that moves axially in both directions, said rack (14) being fitted at one end to a third perpendicular extension.

3. An improved retractor according to claim 1, **characterised in that** the elongated clamshell shaped extensions (9 and 11) are made from a body that has an upper portion that is adjustable to the elongated arms (1 and 2) and to the central support element (15) and a lower elongated section with an arched shaped arrangement.

4. An improved retractor according to claims 1 and 3, **characterised in that** the stated upper section of the side elongated clamshell shaped extensions (9) has a through opening that opens up to the inner side wall of the lower section.

5. An improved retractor according to claim 1, **characterised in that** the bridging element (10) has some means of fixing an adjustable surgical support.

6. An improved retractor according to claim 2, **characterised in that** the toothed wheel (13) has some means of locking to prevent the turning movement of same.

7. An improved retractor according to claims 1 and 4, **characterised in that** it has some means of illumination fitted for the working zone that can be inserted by means of the through openings (16) provisioned in the upper section of the side elongated clamshell like extensions (9).

8. An improved retractor according to claim 1, **characterised in that** the bridging element (10) is fixed to both elongated sections (7) of each one of the arms (1 and 2) by means of side flanges.
